# EUROPEAN PATENT APPLICATION

(11) **EP 3 521 819 A1**
(43) Date of publication of application: **07.08.2019**
(21) Application number: 18155343.9
(22) Date of filing: 06.02.2018
(51) Int. Cl.: G01N 33/00

(54) **OZONE AND/OR NITROGEN DIOXIDE SENSING APPARATUS**

(71) Applicant: Airlabs BV, 1082 MS Amsterdam (NL)
(72) Inventor: Johnson, Matthew, 22647 Lund (SE); Meusinger, Carl, 2200 COPENHAGEN (DK); Russell, Hugo Savill, Bristol BS6 7NT (GB); Gaik, Bartosz, 41200 Sosnowiec (PL); Schmidt, Johan Albrecht, 2200 Copenhagen (DK); Andersen, Mads, 2100 Copenhagen (DK); Yu, Weijia, FDK 2100 Copenhagen (DK); Shen, Yuwei, Beijing, Haidian (CN)
(74) Representative: Kjerrumgaard, Lars Bo

(57) **Abstract**

The present invention relates to a sensing apparatus for monitoring and/or measuring concentrations of ozone and/or nitrogen dioxide in air comprising a chamber (18) having an inlet (12) and an outlet (14), a gas sensor (30) located inside the chamber (18), at least one ozone filter (40) or combined ozone and nitrogen dioxide filter located outside the chamber (18) and in air tight connection with the chamber, and pumping means (20, 22) adapted for moving air from the chamber (18) out through the outlet (14) and for recirculating air from the filter (40) into the chamber (18).

## Description

### FIELD OF THE INVENTION

The present invention relates to an apparatus for monitoring and/or measuring concentrations of a specific component of a gas stream. Moreover, the present invention concerns use of such a sensing apparatus as well as a method of operating such a system. Furthermore, the present invention concerns a novel combined ozone and NO₂ filter which may be used in the sensing apparatus of the present invention.

### BACKGROUND OF THE INVENTION

According to the World Health Organization, 3 million people die every year due to exposure to ambient (outdoor) air pollution, and 92% of the world's population lives in places where air quality exceeds WHO guideline limits. In addition, 4.3 million deaths a year result from indoor air pollution.

The WHO sets guideline limits for four pollutants, particulate matter (PM), ozone (O₃), nitrogen dioxide (NO₂) and sulfur dioxide (SO₂). The U. S. Environmental Protection Agency has a similar list of 'criteria air pollutants': CO, Pb, O₃, PM, NO₂ and SO₂. There is therefore a need for monitoring air pollution as a matter of public health. Unfortunately monitoring stations are few and far between due to the high cost of monitoring equipment. These few stations cannot give an accurate description of human exposure in locations such as inside of buildings, in traffic, on a busy street, inside a bus or subway or in a train station. Inexpensive monitors are available which may be based on electrochemical sensors or on semiconducting sensors, however they are known to have significant problems with cross sensitivity between different components of air, baseline drift and reliability, making the use and interpretation of results difficult. Nonetheless, there is a persistent vision of creating pollution monitoring networks in cities using many cheap sensor based monitors as part of the internet of things (IOT). In addition inexpensive monitors would find use in control systems for building ventilation systems, air management infrastructure, personal exposure monitoring, to name a few.

### SUMMARY OF THE INVENTION

Here we describe a new invention that is able to overcome the traditional problems that have held back low-cost air pollution monitors. The sensing apparatus and use hereof allows low cost yet accurate monitoring of ozone and NO₂ at the same time. The present invention can be extended to cover other gasses such as SO₂ and chlorine.

The present invention described herein is unique in that it uses three or more gas paths in order to overcome inherent limitations of prior art gas sensing apparatuses including for instance small and inexpensive semiconductor and electrochemical sensors. The invention described here recirculates air in a sample chamber generating a transient that can be analyzed for additional information.

In one aspect the present invention concerns a sensing apparatus for monitoring and/or measuring concentrations of a specific component of a gas stream comprising:
a) a chamber having an inlet and an outlet,
b) a gas sensor located inside the chamber,
c) at least one filter for removing and/or trapping the specified component, wherein the filter is located outside the chamber and in gas tight connection with the chamber,
d) a pumping means adapted for moving gas from the chamber and out through the outlet, and/or optionally for recirculating gas from the chamber to the filter,
e) an optional second pumping means adapted for recirculating gas from the filter into the chamber,
wherein the apparatus is adapted to operate when current is applied,
wherein the gas sensor is adapted to provide an output signal to be processed and presented to a user interface.

In a second aspect the present invention relates to a sensing apparatus for monitoring and/or measuring concentrations of ozone and/or nitrogen dioxide in air comprising:
a) a chamber having an inlet and an outlet,
b) a gas sensor located inside the chamber,
c) at least one ozone filter or combined ozone and nitrogen dioxide filter located outside the chamber and in air tight connection with the chamber,
d) a pumping means adapted for moving air from the chamber and out through the outlet, and/or optionally for recirculating air from the chamber to the filter,
e) an optional second pumping means adapted for recirculating air from the filter into the chamber,
wherein the apparatus is adapted to operate when current is applied,
wherein the gas sensor is adapted to provide an output signal to be processed and presented to a user interface.

In an embodiment of the second aspect the sensing apparatus comprising:
a) a chamber having an inlet and an outlet,
b) a gas sensor located inside the chamber,
c) at least one ozone filter or combined ozone and nitrogen dioxide filter located outside the chamber and in air tight connection with the chamber,
d) a first pumping means adapted for moving air from the chamber and out through the outlet,
e) a second pumping means adapted for recirculating air from the filter into the chamber.

In another embodiment of the second aspect the sensing apparatus wherein no valves are present.

In a further embodiment of the second aspect the sensing apparatus comprising:
a) a chamber having an inlet and an outlet,
b) a gas sensor located inside the chamber,
c) at least one ozone filter or combined ozone and nitrogen dioxide filter located outside the chamber and in air tight connection with the chamber,
d) a pumping means adapted for moving air from the chamber and out through the outlet, and for recirculating air from the chamber to the filter. In an embodiment a first valve is present at the inlet to control air flow into the inlet, and a second valve is present and located in a connection line between the pumping means and the at least one filter, to control air flow out of the outlet or recirculating air flow to the at least one filter.

In a further embodiment of the second aspect the sensing apparatus comprising:
a) a chamber having an inlet and an outlet,
b) a gas sensor located inside the chamber,
c1) a first ozone filter or combined ozone and nitrogen dioxide filter located outside the chamber and in air tight connection with the chamber,
c2) a second ozone filter or combined ozone and nitrogen dioxide filter located outside the chamber and in air tight connection with the chamber,
d) a first pumping means adapted for moving air from the chamber and out through the outlet,
e1) a second pumping means adapted for recirculating air from the first filter into the chamber,
e2) a third pumping means adapted for recirculating air from the second filter into the chamber.

In a further embodiment of the sensing apparatus, the output signal from the gas sensor is adapted to be processed by a device for measuring voltage, such as an analog digital converter.

In a still further embodiment of the sensing apparatus, the user interface is selected form any one of a computer, micro controller unit, an ipad, a smart phone, a notebook, and a mac, incl monitor, as well as any other apparatus that makes it possible for a user to follow the process parameters of the sensing apparatus when in operation.

In a further embodiment of the sensing apparatus, the user interface is an integrated part of the sensing apparatus.

In a still further embodiment of the sensing apparatus it is a portable stand-alone apparatus.

In a further embodiment of the sensing apparatus, the at least one filter is an ozone filter.

In a still further embodiment of the sensing apparatus the at least one filter is a combined ozone and nitrogen dioxide filter.

In a further embodiment of the sensing apparatus, the gas sensor is selected from any one of a semiconducting metal oxide material (metal oxides include SnO₂, WO₃, In₂O₃, ZnO), an electrochemical cell sensor, and a photometric sensor.

In a still further embodiment of the sensing apparatus, the first filter is an ozone filter.

In a further embodiment of the sensing apparatus the first filter is a combined ozone and nitrogen dioxide filter.

In a still further embodiment of the sensing apparatus, the first filter is an ozone filter and the second filter is a combined ozone and nitrogen dioxide filter.

In a further embodiment of the sensing apparatus, the ozone filter is selected from a MnO₂ and long chained unsaturated hydrocarbons (e.g. beta-carotene or oleic acid).

In a still further embodiment of the sensing apparatus, the combined ozone and nitrogen dioxide filter is selected from a chemical scrubber filter, active carbon, alkaline carbon, Metal Organic Framework (MOF, e.g. Aluminium Fumarate, Cu-BTC, Fe-BTC) and other graphene/carbon-based materials, catalysts (e.g. gold nanoclusters, metal oxides), and photocatalysis (e.g. titanium dioxide, mixed metal oxides, composite metal oxide/graphene).

In a still further embodiment the filter is a light source for example an LED, who's action changes the relative abundances of NO, NO₂ and ozone, combined with a filter that removes ozone.

In a further aspect the present invention relates to a method for monitoring and/or measuring concentrations of ozone and/or nitrogen dioxide in air comprising using a sensing apparatus of any one of the above embodiments and supply current.

In an embodiment of the method the current is supplied by means of a battery.

In a further aspect the present invention relates to a combined ozone and nitrogen dioxide filter comprising an ozone filter, a photochemical treatment zone and light-emitting diode (LED), wherein the ozone filter is adjacent the photochemical treatment zone and the LED provides energy to the photochemical treatment zone.

The present invention provides these advantages with the described solution.

Further objects and advantages of the present invention will appear from the following description, and claims.

### DESCRIPTION OF THE INVENTION

A monitoring apparatus for O₃, NO₂, SO₂ and other oxidizing gases that can compensate for the inherent limitations of inexpensive sensors, such as electrochemical and metal oxide semiconductor based sensors, has been developed. The apparatus circulates air through filters that have different interactions with the target gases, for example two filters, one an MnO₂ catalyst affecting ozone and another a chemical filter that selectively destroys NO₂ and ozone. One additional type of filter would be a photochemical filter. The first filter removes ozone, the second filter removes both ozone and NO₂. The invention concerns the entire process whereby concentrations of the gases can be determined by recalibrating the 'unreliable' sensor by alternating gas flows through the different filters, or, through no filter at all. In addition, the invention concerns the topology of the gas flow and the design of the filter elements themselves. The additional information provided by the sensor's response to the different air streams results in a significant improvement in system performance including accuracy, precision and long-term stability. In addition, the system may be used in conjunction with temperature modulation of the sensor to further refine the response.

In one aspect the present invention concerns a sensing apparatus for monitoring and/or measuring concentrations of a specific component of a gas stream comprising:
a) a chamber having an inlet and an outlet,
b) a gas sensor located inside the chamber,
c) at least one filter for removing and/or trapping the specified component, wherein the filter is located outside the chamber and in gas tight connection with the chamber,
d) a pumping means adapted for moving gas from the chamber and out through the outlet, and/or optionally for recirculating gas from the chamber to the filter,
e) an optional second pumping means adapted for recirculating gas from the filter into the chamber,
wherein the apparatus is adapted to operate when current is applied,
wherein the gas sensor is adapted to provide an output signal to be processed and presented to a user interface.

Such a gas stream can be of any origin and may for instance be air, such as ambient air (e.g. city air, indoor air, air in underground facilities such as subways, hospital air, air in schools, and air in public spaces), and can also be natural gas, an exhaust stream from industry, atmosphere in a mine or a factory, biogas, combustion exhaust, and exhaust gas from livestock, food and industrial production, exhaust from point sources of pollution, and air in enclosed, semi-enclosed and open spaces.

The "specific component" of a gas stream as used herein is one or more components present in the gas stream to be monitored or measured, such as O₃, NO₂, SO₂, Cl₂ and other oxidizing gases, volatile organic compounds (VOC), ammonia and other reducing gases such as amines, reduced sulfur compounds including hydrogen sulfide, organic sulfides and thiols,.

In a second aspect the present invention relates to a sensing apparatus for monitoring and/or measuring concentrations of ozone and/or nitrogen dioxide in air comprising:
a) a chamber having an inlet and an outlet,
b) a gas sensor located inside the chamber,
c) at least one ozone filter or combined ozone and nitrogen dioxide filter located outside the chamber and in air tight connection with the chamber,
d) a pumping means adapted for moving air from the chamber and out through the outlet, and/or optionally for recirculating air from the chamber to the filter,
e) an optional second pumping means adapted for recirculating air from the filter into the chamber,
wherein the apparatus is adapted to operate when current is applied,
wherein the gas sensor is adapted to provide an output signal to be processed and presented to a user interface.

The chamber can be made of any suitable material, such as steel, polymers such as thermoplastic, fluoropolymers, polyethylene, glass or ceramic and gas or air is led through the inlet and out through the outlet and through any of the connections between the pumps and/or the filters in an air tight way, meaning that all such connections are made of material that does not decompose under the given circumstances and parameters.

The gas sensor is mounted inside the chamber to be accessible to air or gas entering the chamber. The gas sensor can be placed in the center of the chamber or adjacent or closer to the sides of the chamber as long as the air or gas stream is allowed to flow freely in and out of the chamber through the various connections, including inlet and outlet.

When the ozone filter or combined ozone and nitrogen dioxide filter or filter for removing and/or trapping the specified component are located outside the chamber and in air tight connection with the chamber, such air tight connection can be plastic tubes or metal tubes, and in order to minimize the size of the apparatus it may be suitable to make the connection as short as possible, such as a small channel from the chamber to the filter.

The pumping means adapted for moving air from the chamber and out through the outlet, and/or optionally for recirculating air from the chamber to the filter, is usually a pump, such as a vacuum pump, peristaltic pump, membrane or niston pump, but mav also be a fan for example a radial or axial fan capable of pushing gas or air through the system Further, pumping means may be present and part of the apparatus, in particular if more filters are connected to the chamber.

The sensing apparatus of the present invention can have any size, but ideally it could be very small, in particular compared to other sensing apparatus known in the prior art, and which do not have the same accuracy when it comes to monitoring and measuring specific components of a gas stream, such as ambient air, wherein ozone and nitrogen dioxide are desired to measure as accurate as possible. I order for the apparatus to function, current should be applied and although a simple plug-in connection to a power outlet may be used, it is preferred to supply current by battery power.

In order to monitor and/or measure specific components of a gas stream, in particular ambient air, wherein ozone and nitrogen dioxide are the components to be measured and/or monitored, the gas sensor is adapted to provide an output signal to be processed and presented to a user interface. The output signal can be via a wire connection from the gas sensor to an analog-digital converter or other suitable device for measuring voltage or current, such as a meter or an analogue to digital converter and then be processed and displayed in a user interface, such as a computer. In principle such signal from the gas sensor can be stored in a storage unit in the sensing apparatus and later be processed and submitted to the user interface.

The term "user interface" as used herein means a computer, an ipad, a smart phone, a notebook, a micro controller unit, and a mac, incl monitor, as well as any other apparatus that makes it possible for a user to follow the process parameters of the device when in operation. The user interface is preferably an integrated part of the device of the present invention but may also be remote.

In an embodiment of the second aspect the sensing apparatus comprises:
a) a chamber having an inlet and an outlet,
b) a gas sensor located inside the chamber,
c) at least one ozone filter or combined ozone and nitrogen dioxide filter located outside the chamber and in air tight connection with the chamber,
d) a first pumping means adapted for moving air from the chamber and out through the outlet,
e) a second pumping means adapted for recirculating air from the filter into the chamber.

In another embodiment of the second aspect the sensing apparatus no valves are present.

In a further embodiment of the second aspect the sensing apparatus comprises:
a) a chamber having an inlet and an outlet,
b) a gas sensor located inside the chamber,
c) at least one ozone filter or combined ozone and nitrogen dioxide filter located outside the chamber and in air tight connection with the chamber,
d) a pumping means adapted for moving air from the chamber and out through the outlet, and for recirculating air from the chamber to the filter.

In an embodiment a first valve is present at the inlet to control air flow into the inlet, and a second valve is present and located in a connection line between the pumping means and the at least one filter, to control air flow out of the outlet or recirculating air flow to the at least one filter. The first valve maybe a one-way valve or a two way valve and the second valve may be a three way valve.

In a further embodiment of the second aspect the sensing apparatus comprising:
a) a chamber having an inlet and an outlet,
b) a gas sensor located inside the chamber,
c1) a first ozone filter or combined ozone and nitrogen dioxide filter located outside the chamber and in air tight connection with the chamber,
c2) a second ozone filter or combined ozone and nitrogen dioxide filter located outside the chamber and in air tight connection with the chamber,
d) a first pumping means adapted for moving air from the chamber and out through the outlet,
e1) a second pumping means adapted for recirculating air from the first filter into the chamber,
e2) a third pumping means adapted for recirculating air from the second filter into the chamber.

Typically, there is one first filter and one second filter but more filters may be applied such as two first filters and/or two second filters.

In a further embodiment of the sensing apparatus the output signal from the gas sensor is adapted to be processed by a device for measuring voltage or current, such as a meter or analog digital converter.

In a still further embodiment of the sensing apparatus the user interface is selected form any one of a computer, micro controller unit, an ipad, a smart phone, a notebook, a PC. Each of the user interfaces are contemplated to be embodiments of the present invention.

In a further embodiment of the sensing apparatus the user interface is an integrated part of the sensing apparatus.

In a still further embodiment of the sensing apparatus it is a portable stand-alone apparatus.

The term "portable stand-alone" as used herein means an apparatus which can be transported by the user and optionally plugged-in at any desired place, to measure and/or monitor components of the gas stream such as ozone and NO₂ of air.

In a further embodiment of the sensing apparatus the at least one filter is an ozone filter.

In a still further embodiment of the sensing apparatus the at least one filter is a combined ozone and nitrogen dioxide filter.

In a further embodiment of the sensing apparatus the gas sensor is selected from any one of a semiconducting metal oxide material, an electrochemical cell sensor, and a photometric sensor. Typically, metal oxides are selected from one or more of SnO₂, WO₃, In₂O₃, and ZnO.

In a still further embodiment of the sensing apparatus the first filter is an ozone filter.

In a further embodiment of the sensing apparatus the first filter is a combined ozone and nitrogen dioxide filter.

In a still further embodiment of the sensing apparatus the first filter is an ozone filter and the second filter is a combined ozone and nitrogen dioxide filter.

In a further embodiment of the sensing apparatus the ozone filter is selected from a MnO₂ filter, an unsaturated hydrocarbon, or graphene-based filter. Typical, unsaturated hydrocarbons are selected from beta-carotene, oleic acid or similar. In a typical embodiment the ozone filter is selected from a MnO₂ filter.

In a still further embodiment of the sensing apparatus, the combined ozone and nitrogen dioxide filter is selected from a photocatalyst, a chemical scrubber filter, active carbon, alkaline carbon, Metal Organic Framework (MOF), and other graphene/carbon-based materials, catalysts, and photolytic filters. Typically, MOF is selected from Aluminium Fumarate, Cu-BTC, and Fe-BTC. Typically, catalysts are selected from metallic molybdenum, gold nanoclusters, metal oxides, and silicon dioxide. Typically, photocatalyst are selected from titanium dioxide, mixed metal oxides, and composite metal oxide/graphene.

In a further aspect the present invention relates to a method for monitoring and/or measuring concentrations of ozone and/or nitrogen dioxide in air comprising using a sensing apparatus of any one of the above embodiments and supply current. In one embodiment the present invention relates to a method for monitoring concentrations of ozone and/or nitrogen dioxide in air comprising using a sensing apparatus of any one of the above embodiments and supply current. In another embodiment the present invention relates to a method for measuring concentrations of ozone and/or nitrogen dioxide in air comprising using a sensing apparatus of any one of the above embodiments and supply current.

In an embodiment of the method the current is supplied by means of a battery.

In a further aspect the present invention relates to a combined ozone and nitrogen dioxide filter comprising an ozone filter, a photochemical treatment zone and light-emitting diode (LED), wherein the ozone filter is adjacent the photochemical treatment zone and the LED provides energy to the photochemical treatment zone, wherein the combined filter is adapted to first receive gas or air comprising ozone and NO₂ in the photochemical treatment zone and then for the irradiated gas or air to enter the ozone filter. The combined filter is supposed to work in the way that gas or air is irradiated with LED light, such as 400 nm LED, before being passed through a short ozone filter. Thus, in an embodiment the LED light is adapted to provide sufficient irradiation of the photochemical treatment zone to convert NO₂ to ozone. The LED light promotes photodissociation of NO₂ that produces ozone, which ozone is then removed. In a further embodiment the LED light is 300 to 500 nm, such as 400 nm.

In a still further aspect the present invention relates to use of a combined ozone and nitrogen dioxide filter comprising an ozone filter, a photochemical treatment zone and light-emitting diode (LED), wherein the ozone filter is adjacent the photochemical treatment zone and the LED provides energy to the photochemical treatment zone, wherein the combined filter is adapted to first receive gas or air comprising ozone and NO₂ in the photochemical treatment zone and then for the irradiated gas or air to enter the ozone filter, for monitoring and/or measuring concentrations of ozone.

The invention will now be described more fully with reference to the appended drawings illustrating typical embodiments of the street furniture with air cleaning devices integrated therein.

These drawings by no means limit the scope of the present invention and are only intended to guide the skilled person for a better understanding of the present invention.

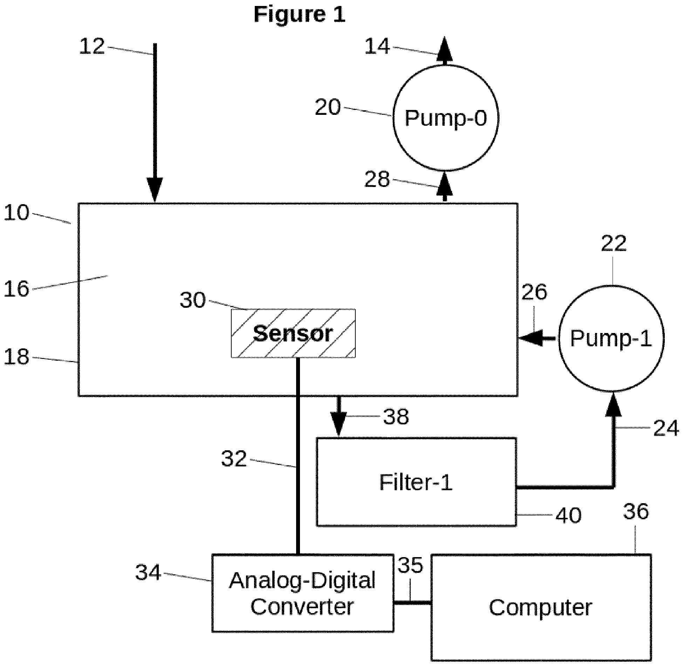

Figure 1 illustrates an embodiment of the sensing apparatus (10) of the present invention, having a chamber (18) within which a gas sensor (30) is located. Gas or air is led through the inlet (12) by means of a pump (20), and into the space (16) of the chamber (18) wherein the gas or air will come in contact with the gas sensor (30). The pump (20) is located at the outlet (14) of the chamber (18) so that gas or air is drawn through a connection (28) and via the pump (20) is transported out of the apparatus (10) at outlet (14). The gas sensor (30) is in communication, such as a wire (32) with an analog digital converter (34) for measuring voltage. The measured voltage is then led to a computer (36) via a communication (35), such as a wire, where it is processed to provide a readable output that can be used to monitoring and/or measuring concentrations of a specific component of a gas stream, such as air. The chamber (18) is furthermore in communication (38) with a filter (40), such as an ozone filter, wherein the gas or air in the chamber (18) is drawn through the filter (40) by means of a second pump (22) through a connection (24), and the filtered gas or air is then led into the chamber (18) through a connection (26). The chamber (18), when in operation, will contain inlet gas or air in mixture with filtered gas or air, which is then drawn out via pump (20) at the outlet (14). This sensing apparatus (10) can operate when current is applied, such as by battery power or connection to a power outlet (not shown).

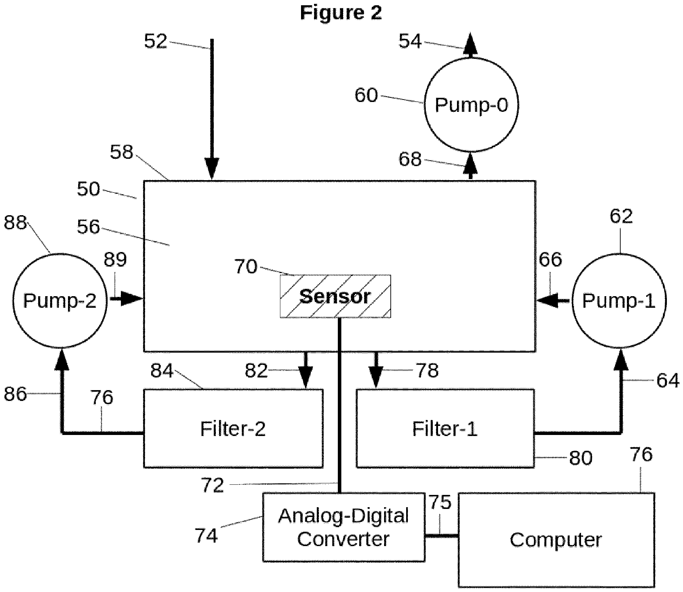

Figure 2 illustrates a preferred embodiment of the sensing apparatus (50) of the present invention, having a chamber (58) wherein a gas sensor (70) is located. Gas or air is led through the inlet (52) by means of a pump (20), and into the space (56) of the chamber (58) wherein the gas or air will come in contact with the gas sensor (70). The pump (60) is located at the outlet (54) of the chamber (58) so that gas or air is drawn through a connection (68) and via the pump (60) is transported out of the apparatus (50) at outlet (54). The gas sensor (70) is in communication, such as a wire (72) with an analog digital converter (74) for measuring voltage. The measured voltage is then led to a computer (76) via a communication (75), such as a wire, where it is processed to provide a readable output that can be used to monitoring and/or measuring concentrations of a specific component of a gas stream, such as air. The chamber (58) is furthermore in communication (78) with a first filter (80), such as an ozone filter, wherein the gas or air in the chamber (58) is drawn through the filter (80) by means of a second pump (62) through a connection (64), and the filtered gas or air is then led into the chamber (58) through a connection (66). The chamber (58) is furthermore in communication (82) with a second filter (84), such as a combined ozone nitrogen dioxide filter, wherein the gas or air in the chamber (58) is drawn through the filter (84) by means of a third pump (88) through a connection (86), and the filtered gas or air is then led into the chamber (58) through a connection (89). The chamber (58), when in operation, will contain inlet gas or air in mixture with filtered gas or air, which is then drawn out via pump (60) at the outlet (54). This sensing apparatus (50) can operate when current is applied, such as by battery power or connection to a power outlet (not shown).

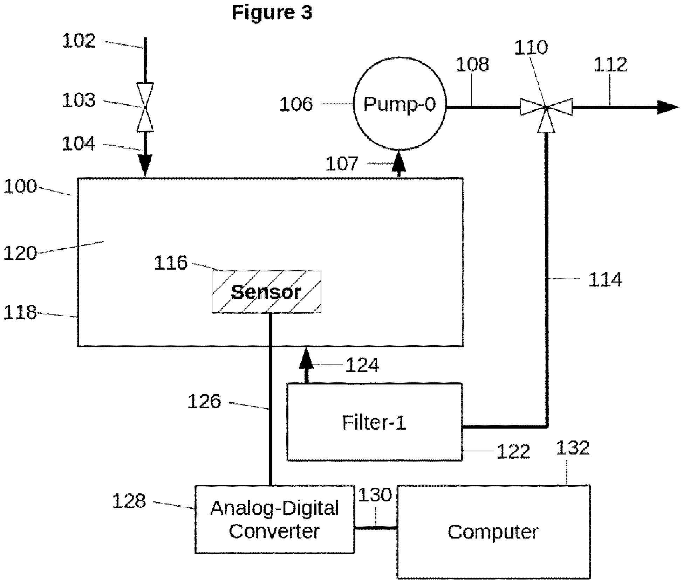

Figure 3 illustrates an embodiment of the sensing apparatus (100) of the present invention, having a chamber (118) wherein a gas sensor (116) is located. Gas or air is led through the inlet (102) by means of a pump (106), and into the space (120) of the chamber (118) wherein the gas or air will come in contact with the gas sensor (116). A first valve (103), typically a two way valve, is located in the inlet (102) to control the flow of gas or air into the chamber (118) via connection (104). The pump (106) is located at the outlet (112) of the chamber (118) so that gas or air is drawn through a connection (107) and via the pump (106) is transported to a second valve (110), typically a three-way valve, and out of the apparatus (100) at outlet (112). The second valve (110) is furthermore in connection (114) with a filter (122), such as an ozone filter, so that gas or air can be transported through the connection (114) and into the filter (122) and further from the filter (122) through a connection (124) into the chamber (118), when the apparatus (100) is in operation. The gas sensor (116) is in communication, such as a wire (126) with an analog digital converter (128) for measuring voltage. The measured voltage is then led to a computer (132) via a communication (130), such as a wire, where it is processed to provide a readable output that can be used to monitoring and/or measuring concentrations of a specific component of a gas stream, such as air. This sensing apparatus (100) can operate when current is applied, such as by battery power or connection to a power outlet (not shown).

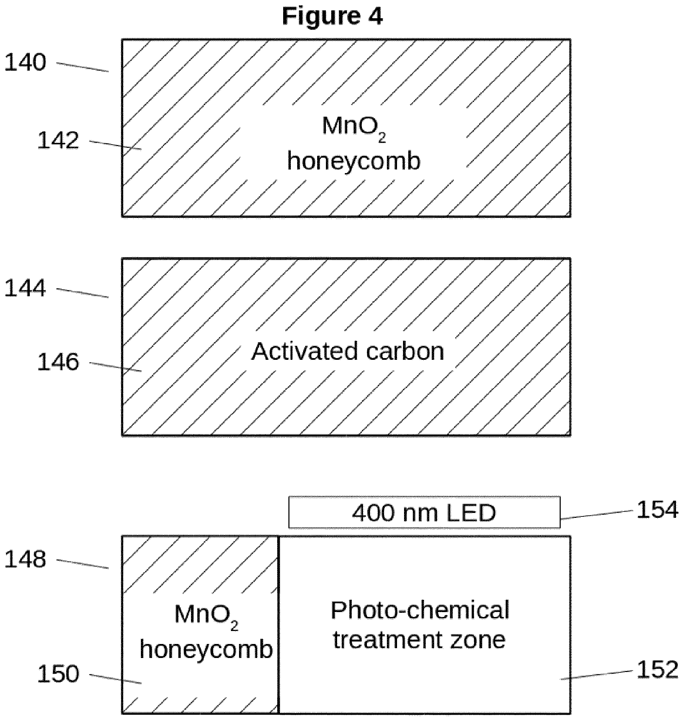

Figure 4 illustrates three embodiments (140, 144, 148) of a filter of the present invention. The first filter (140) is an ozone filter to be used as the filter in the apparatus of the present invention. The ozone filter is a MnO₂ honeycomb filter (142). Such a filter (142) is very useful in the apparatus of the present invention where there is one filter or in the situation where there are two filters, and this is one of the filters. Another filter (144) is a combined ozone and nitrogendioxide filter to be used as the filter in the apparatus of the present invention. The combined filter is an activated carbon filter (146). Such a filter (146) is very useful in the apparatus of the present invention where there is one filter or in the situation where there are two filters, and this is one of the filters. In that respect one filter may be the ozone filter (142) and the second filter is the combined filter (146). A further filter (148) which is a combined ozone and nitrogen dioxide filter also constitutes an invention. The filter (148) is composed of a MnO₂ honeycomb filter (150) adjacent a combined ozone and NO₂ filter wherein a photochemical treatment zone (152) is adapted to receive a gas or air and the gas or air is then exposed to LED light (154) with a wavelength of for instance 400 nm.

All references, including publications, patent applications and patents, cited herein are hereby incorporated by reference to the same extent as if each reference was individually and specifically indicated to be incorporated by reference and was set forth in its entirety herein.

All headings and sub-headings are used herein for convenience only and should not be construed as limiting the invention in any way.

Any combination of the above-described elements in all possible variations thereof is encompassed by the invention unless otherwise indicated herein or otherwise clearly contradicted by context.

Recitation of ranges of values herein are merely intended to serve as a short method of referring individually to each separate value falling within the range, unless other-wise indicated herein, and each separate value is incorporated into the specification as if it were individually recited herein. Unless otherwise stated, all exact values provided herein are representative of corresponding approximate values (e.g., all exact exemplary values provided with respect to a particular factor or measurement can be considered to also provide a corresponding approximate measurement, modified by "about", where appropriate).

All methods described herein can be performed in any suitable order unless other-wise indicated herein or otherwise clearly contradicted by context.

The terms "a" and "an" and "the" and similar referents as used in the context of describing the invention are to be construed to insert both the singular and the plural, unless otherwise indicated herein or clearly contradicted by context. Thus, "a" and "an" and "the" may mean at least one, or one or more.

The term "and/or" as used herein means each individual alternative as well as the combined alternatives, for instance, "for monitoring and/or measuring concentrations" is intended to mean for monitoring alone, for measuring alone, or both monitoring and measuring concentrations at the same time.

The use of any and all examples, or exemplary language (e.g., "such as") provided herein, is intended merely to better illuminate the invention and does not pose a limitation on the scope of the invention unless otherwise indicated. No language in the specification should be construed as indicating any element is essential to the practice of the invention unless as much is explicitly stated.

Throughout the description when "selected from" or "selected from the group consisting of" is used it also means all possible combinations of the stated terms, as well as each individual term.

The citation and incorporation of patent documents herein is done for convenience only and does not reflect any view of the validity, patentability and/or enforceability of such patent documents.

The description herein of any aspect or embodiment of the invention using terms such as "comprising", "having", "including" or "containing" with reference to an element or elements is intended to provide support for a similar aspect or embodiment of the invention that "consists of", "consists essentially of", or "substantially comprises" that particular element or elements, unless otherwise stated or clearly contradicted by context (*e.g.,* a composition described herein as comprising a particular element should be understood as also describing a composition consisting of that element, unless otherwise stated or clearly contradicted by context).

The features disclosed in the foregoing description may, both separately and in any combination thereof, be material for realizing the invention in diverse forms thereof.

### EXPERIMENTALS

Referring to Figure 2 air flows into a sampling chamber (58) via Polytetrafluoroethylene (PTFE, also known as "Teflon") tubes (52) and an air pump (60) and a flow rate of 0.5 to 3 L/min. The air pump is placed at the end of sampling line (68), right before the exhaust (54), to prevent contamination of the sample air by the pump. The sample chamber is built from PTFE, stainless steel or other inert material. The inlet line (52) is connected to one side of the sample chamber and the outlet line is connected to the other side. The sample chamber has an inner volume between 0.01 and 0.5 L. The gas sensor (70) is mounted inside the sample chamber together other sensors including but not limited to temperature, humidity, and pressure sensor(s). Electronic wires (72) connected to the sensors exit the sample chamber via e.g. a cable gland insuring an air tight seal. The recirculating filter 1 (80 in figure 2) consists of a filter house connected to a small air pump (88). The filter house contains an ozone catalyst (specifically MnO₂ coated PTFE beads, or MnO₂ coated honeycomb mesh). The filter house is compact (e.g. 30mmx10mmx10mm) and flow through the filter. The recirculating filter 2 (84 in figure 2) removes both ozone and NO₂. This can be achieved by photochemical filter, where air in the filter house is irradiated with 400 nm LED light before being passed through a short ozone filter. The light promotes photodissociation of NO₂ that produces ozone, the produced is then removed. This leads to an overall reduction in the level of NO₂ and ozone (while level of NO may increase).

The filters need not be 100 % efficient. As air is being recirculated, this creates a transient in the sensor response that is directly related to the concentration of ozone and NO₂ in the sample air. This provides for better more accurate measurements of ozone and NO₂ free from interferences. We have built an example of the sensor and characterized its performance.

### Description of sensor:

The sensor shown in diagram is the device sensitive to the concentration of gases in air. Gases include ozone, NO₂, Cl₂, SO₂, and other. The sensor could be an electrochemical type gas sensor or a semiconducting metal oxide type gas sensor (metal oxides include SnO₂, WO₃, In₂O₃, ZnO and other). In both cases the sensor is probed by a device the for measuring voltage or resistance, such as an analog-digital converter. This device is connected to computer that performs relevant analysis of data.

### Description of valve-based system:

Alternatively, the system can also be operated in valve-based setup. This setup only requires 1 pump (see Fig. 3). During normal operation, Valve-1 will be open and Valve-2 will direct air from Pump-0 to Outlet. During recirculation operation Valve-1 is closed and and Valve-2 is set to direct air from Pump-0 to Filter-1. The system can be extended to include more filters by adding more valves.

### Dimensions of the invention:

The system is portable. The system can be fitted in case smaller than 150mm x 200mm x 100mm. The weight of the system can be smaller than 1 kg. The system can be powered by battery.

### List of possible filters/filter materials:

### Filter for selectively removing ozone:

MnO₂ (honeycomb, pellets, or other media), long chained alkenes (e.g. beta-carotene).

### Filter for removing ozone and NO₂:

Active carbon, alkaline carbon, metals (e.g. Molybdenum), Metal Organic Framework (MOF, e.g. Aluminium Fumarate, Cu-BTC, Fe-BTC) and other graphene/carbon-based materials, catalysts (e.g. gold nanoclusters, metal oxides), and photocatalysis (e.g. titanium dioxide, mixed metal oxides, composite metal oxide/graphene).

## Claims

1. A sensing apparatus for monitoring and/or measuring concentrations of a specific component of a gas stream comprising:
a) a chamber having an inlet and an outlet,
b) a gas sensor located inside the chamber,
c) at least one filter for removing and/or trapping the specified component, wherein the filter is located outside the chamber and in gas tight connection with the chamber,
d) a pumping means adapted for moving gas from the chamber and out through the outlet, and/or optionally for recirculating gas from the chamber to the filter,
e) an optional second pumping means adapted for recirculating gas from the filter into the chamber,
wherein the apparatus is adapted to operate when current is applied,
wherein the gas sensor is adapted to provide an output signal to be processed and presented to a user interface.

2. A sensing apparatus for monitoring and/or measuring concentrations of ozone and/or nitrogen dioxide in air comprising:
a) a chamber having an inlet and an outlet,
b) a gas sensor located inside the chamber,
c) at least one ozone filter or combined ozone and nitrogen dioxide filter located outside the chamber and in air tight connection with the chamber,
d) a pumping means adapted for moving air from the chamber and out through the outlet, and/or optionally for recirculating air from the chamber to the filter,
e) an optional second pumping means adapted for recirculating air from the filter into the chamber,
wherein the apparatus is adapted to operate when current is applied,
wherein the gas sensor is adapted to provide an output signal to be processed and presented to a user interface.

3. The sensing apparatus of claim 2 comprising:
a) a chamber having an inlet and an outlet,
b) a gas sensor located inside the chamber,
c) at least one ozone filter or combined ozone and nitrogen dioxide filter located outside the chamber and in air tight connection with the chamber,
d) a first pumping means adapted for moving air from the chamber and out through the outlet,
e) a second pumping means adapted for recirculating air from the filter into the chamber.

4. The sensing apparatus of claim 2 wherein no valves are present.

5. The sensing apparatus of claim 2 comprising:
a) a chamber having an inlet and an outlet,
b) a gas sensor located inside the chamber,
c) at least one ozone filter or combined ozone and nitrogen dioxide filter located outside the chamber and in air tight connection with the chamber,
d) a pumping means adapted for moving air from the chamber and out through the outlet, and for recirculating air from the chamber to the filter.

6. The sensing apparatus of claim 5 wherein a first valve is present at the inlet to control air flow into the inlet, and a second valve is present and located in a connection line between the pumping means and the at least one filter, to control air flow out of the outlet or recirculating air flow to the at least one filter.

7. The sensing apparatus of claim 2 comprising:
a) a chamber having an inlet and an outlet,
b) a gas sensor located inside the chamber,
c1) a first ozone filter or combined ozone and nitrogen dioxide filter located outside the chamber and in air tight connection with the chamber,
c2) a second ozone filter or combined ozone and nitrogen dioxide filter located outside the chamber and in air tight connection with the chamber,
d) a first pumping means adapted for moving air from the chamber and out through the outlet,
e1) a second pumping means adapted for recirculating air from the first filter into the chamber,
e2) a third pumping means adapted for recirculating air from the second filter into the chamber.

8. The sensing apparatus of any one of the preceding claims wherein the output signal from the gas sensor is adapted to be processed by a device for measuring voltage, such as an analog digital converter.

9. The sensing apparatus of any one of the preceding claims wherein the user interface is selected form any one of a computer, micro controller unit, an ipad, a smart phone, a notebook, and a mac, incl monitor, as well as any other apparatus that makes it possible for a user to follow the process parameters of the sensing apparatus when in operation.

10. The sensing apparatus of any one of the preceding claims wherein the user interface is an integrated part of the sensing apparatus.

11. The sensing apparatus of any one of the preceding claims wherein it is a portable stand-alone apparatus.

12. The sensing apparatus of any one of the preceding claims wherein the at least one filter is an ozone filter.

13. The sensing apparatus of any one of the preceding claims wherein the at least one filter is a combined ozone and nitrogen dioxide filter.

14. The sensing apparatus of any one of the preceding claims wherein the gas sensor is selected from any one of a semiconducting metal oxide material, an electrochemical cell sensor, and a photometric sensor.

15. The sensing apparatus of any one of the preceding claims wherein the first filter is an ozone filter.

16. The sensing apparatus of any one of the preceding claims wherein the first filter is a combined ozone and nitrogen dioxide filter.

17. The sensing apparatus of any one of the preceding claims 7-16 wherein the first filter is an ozone filter and the second filter is a combined ozone and nitrogen dioxide filter.

18. The sensing apparatus of any one of the preceding claims wherein the ozone filter is selected from a MnO₂ and long chained alkenes.

19. The sensing apparatus of any one of the preceding claims wherein the combined ozone and nitrogen dioxide filter is selected from a photocatalyst, a chemical scrubber filter, active carbon, alkaline carbon, Metal Organic Framework and other graphene/carbon-based materials, catalysts, and photocatalysis.

20. A method for monitoring and/or measuring concentrations of ozone and/or nitrogen dioxide in air comprising using a sensing apparatus of any one of the claims 1-19 and supply current.

21. The method of claim 20 wherein current is supplied by means of a battery.

22. A combined ozone and nitrogen dioxide filter comprising an ozone filter, a photochemical treatment zone and light-emitting diode (LED), wherein the ozone filter is adjacent the photochemical treatment zone and the LED provides energy to the photochemical treatment zone, wherein the combined filter is adapted to first receive gas or air comprising ozone and NO₂ in the photochemical treatment zone and then for the irradiated gas or air to enter the ozone filter.
